# FASCICULE DE BREVET EUROPEEN

(11) **EP 2 854 714 B1**
(45) Date de publication et mention de la délivrance du brevet: **21.10.2020**
(21) Numéro de dépôt: 13728488.1
(22) Date de dépôt: 24.05.2013
(51) Int. Cl.: A61F 2/44

(54) **IMPLANT VERTÉBRAL DOTÉ D'UN MOYEN AUTONOME DE VERROUILLAGE ET DE DÉVERROUILLAGE**
WIRBELIMPLANTAT MIT SELBSTVERRIEGELUNGS- UND ENTRIEGELUNGSVORRICHTUNG
VERTEBRAL IMPLANT PROVIDED WITH A SELF LOCKING AND UNLOCKING MEANS

(30) Priorité: 25.05.2012 FR 1254831
(43) Date de publication de la demande: 08.04.2015
(73) Titulaire: Spink Up Inc., Miami, Florida 33132 (US)
(72) Inventeur: RICHERME, Pierre, Dominique, F-74120 Praz sur Arly (FR); RAMARE, Stéphane, F-72190 NEUVILLE SUR SARTHE (FR); TAMBURINI, Christophe, F-69720 Saint Laurent de Mure (FR)
(74) Mandataire: Bringer IP
(86) Numéro de dépôt international: PCT/FR2013/051153
(87) Numéro de publication internationale: WO 2013/175147

(56) Documents cités:
- EP-A1- 1 561 429
- EP-A1- 2 319 438
- WO-A1-00/24325
- WO-A1-00/66045
- WO-A1-2009/012195
- WO-A1-2010/019394
- FR-A1- 2 856 272
- US-A1- 2004 260 306
- US-A1- 2011 313 528

## Description

### DOMAINE TECHNIQUE

La présente invention se rapporte au domaine technique et général des systèmes de fixation pour l'ostéosynthèse du rachis et concerne en particulier les implants destinés à être implantés dans l'espace intersomatique d'un patient.

La présente invention concerne plus particulièrement un implant vertébral destiné à être implanté dans l'espace intersomatique d'un patient et comprenant une cage intersomatique et au moins une vis de fixation destinée à être vissée dans la masse osseuse au travers d'un puits de ladite cage pour stabiliser la position de ladite cage au sein de l'espace intersomatique.

L'invention concerne également un implant vertébral destiné à être implanté le long de l'espace intersomatique d'un patient et comprenant une plaque intersomatique et au moins une vis de fixation destinée à être vissée dans la masse osseuse au travers d'un puits de ladite plaque pour stabiliser la position de ladite plaque le long de l'espace intersomatique.

Ces implants ont pour objectif de traiter diverses pathologies du rachis comme des tassements vertébraux, des scolioses, des lordoses, des cyphoses ou des instabilités vertébrales. Ces pathologies sont citées bien évidemment à titre illustratif et non limitatif.

Ces implants se présentent notamment sous la forme de cages intersomatiques. Il s'agit d'implants creux destinés à accueillir un matériel ostéoinducteur, tel qu'un greffon osseux, et sont destinés à être implantés par voie chirurgicale dans l'espace intersomatique vertébral d'un patient, après préparation adéquate, afin d'obtenir une consolidation osseuse ou fusion de deux segments rachidiens. De telles cages peuvent aussi être utilisées en tant que prothèse pour retrouver une hauteur discale appropriée.

Le bon fonctionnement de ces cages repose notamment sur leur maintien en position au cours du temps. Toute migration de la cage au sein de l'espace intersomatique peut avoir des conséquences particulièrement néfastes pour le patient.

Si la cage n'a pas été correctement positionnée lors de l'intervention chirurgicale, elle peut créer un conflit radiculaire immédiat ou être expulsée par la suite à la faveur de micro-mouvements. Il existe également des situations où la pression résiduelle intra discale peut expulser l'implant. Ces situations sont dès lors synonymes de dysfonctionnement de l'implant et peuvent également nécessiter une reprise chirurgicale, ce qui est bien évidemment à proscrire à cause notamment des risques bien connus liés à toute intervention chirurgicale.

Ainsi pour éviter l'ensemble de ces problèmes, toute cage intersomatique doit comporter également des moyens stabilisateurs de sa position. Par ailleurs, dans des cas de fortes migrations ou de problèmes spécifiques liés à l'implant, il est parfois souhaitable de retirer l'implant. Ainsi, ces moyens stabilisateurs doivent idéalement ne pas empêcher le retrait de l'implant dans un cas de force majeure.

### TECHNIQUE ANTERIEURE

Classiquement, certains moyens stabilisateurs des cages intersomatiques sont mis en oeuvre par une instrumentation pédiculaire postérieure et peuvent comporter une plaque orthopédique. Cela se traduit par la pose d'une plaque cervicale ou d'une plaque lombaire qui est installée sur le mur vertébral et tenue par des vis implantées dans les vertèbres.

Une telle solution permet effectivement de stabiliser la position de la cage intersomatique mais présente l'inconvénient d'ajouter une surépaisseur au niveau des murs vertébraux et complique également la pose de la cage en rendant l'intervention chirurgicale plus invasive. En effet, les dimensions de la plaque ainsi que l'éloignement relatif des différentes positions de vissage, situées sur des vertèbres à des étages différents, impliquent une opération chirurgicale relativement conséquente.

Toute solution mettant en œuvre des moyens stabilisateurs peu invasifs permettant de maintenir la cage en position tout en n'empêchant pas son possible retrait constitue dès lors une avancée majeure dans le domaine des implants rachidiens. Les publications WO 00/66045 A1, FR 2 856 272 A1, EP 2 319 438 A1 et EP 1 561 429 A1 présentent des exemples d'implant destinés à être implantés dans ou le long de l'espace intersomatique d'un patient: ceux-ci comprenent une cage intersomatique et des vis de fixation ayant des systèmes escamotables de modification de la circonférence des têtes dedites vis permettant leur verrouillage et leur déverrouillage.

### EXPOSE DE L'INVENTION

Les objets assignés à la présente invention visent en conséquence à remédier aux différents inconvénients énumérés précédemment et à proposer un nouvel implant vertébral dont la position est stabilisée et la mise en œuvre simplifiée.

Un autre objet de l'invention vise à proposer un nouvel implant vertébral aisément implantable au sein de l'espace intersomatique.

Un autre objet de l'invention vise à proposer un nouvel implant vertébral dont les dispositifs de maintien de la position sont mis en oeuvre de manière peu invasive pour le patient.

Un autre objet de l'invention vise à proposer un nouvel implant vertébral dont les moyens de stabilisation sont sûrs et aisément mis en œuvre.

Un autre objet de l'invention vise à proposer un nouvel implant vertébral comportant des moyens de stabilisation et que l'on peut aisément retirer.

Un autre objet de l'invention vise à proposer un nouvel implant vertébral qui permet une ostéosynthèse améliorée.

L'invention vise enfin à proposer un nouveau kit médical permettant d'implanter l'implant vertébral rapidement et de manière efficace.

Les objets assignés à l'invention sont atteints par un implant vertébral destiné à être implanté dans l'espace intersomatique d'un patient et comprenant une cage intersomatique et au moins une vis de fixation destinée à être vissée dans la masse osseuse au travers d'un puits de ladite cage pour stabiliser la position de ladite cage au sein de l'espace intersomatique, ledit implant étant caractérisé en ce que le puits et la vis forment un système de verrouillage autonome de la vis dans le puits, ledit système comprenant un moyen mâle solidaire de la vis et un moyen femelle formé par les parois du puits, lesdits moyens mâle et femelle étant montés avec une flexibilité relative autorisant lors du vissage de la vis dans la masse osseuse au travers du puits, une mise en contrainte progressive et mutuelle des moyens mâle et femelle jusqu'à atteindre une position finale de verrouillage de la vis dans laquelle les moyens mâle et femelle sont mutuellement relâchés, le moyen mâle étant verrouillé en position par le moyen femelle, ladite vis comprenant au moins une tête et un corps principal, ledit moyen femelle étant fixe et ledit moyen mâle étant mobile et comportant un système escamotable de modification de la circonférence de ladite tête permettant lors du vissage la flexibilité relative entre lesdits moyens mâle et femelle

Les objets assignés à l'invention sont atteints par un implant vertébral destiné à être implanté le long de l'espace intersomatique d'un patient et comprenant une plaque intersomatique et au moins une vis de fixation destinée à être vissée dans la masse osseuse au travers d'un puits de ladite plaque pour stabiliser la position de ladite plaque le long de l'espace intersomatique, ledit implant étant caractérisé en ce que le puits et la vis forment un système de verrouillage autonome de la vis dans le puits, ledit système comprenant un moyen mâle solidaire de la vis et un moyen femelle formé par les parois du puits, lesdits moyens mâle et femelle étant montés avec une flexibilité relative autorisant lors du vissage de la vis dans la masse osseuse au travers du puits, une mise en contrainte progressive et mutuelle des moyens mâle et femelle jusqu'à atteindre une position finale de verrouillage de la vis dans laquelle les moyens mâle et femelle sont mutuellement relâchés, le moyen mâle étant verrouillé en position par le moyen femelle, ladite vis comprenant au moins une tête et un corps principal, ledit moyen femelle étant fixe et ledit moyen mâle étant mobile et comportant un système escamotable de modification de la circonférence de ladite tête permettant lors du vissage la flexibilité relative entre lesdits moyens mâle et femelle.

Les objets assignés à l'invention sont également atteints à l'aide d'un kit médical formé par un implant vertébral conforme à l'invention et un ancillaire de mise en place dudit implant vertébral dans l'espace intersomatique d'un patient.

### DESCRIPTIF SOMMAIRE DES DESSINS

D'autres objets et avantages de l'invention apparaîtront mieux à la lecture de la description ci-après, ainsi qu'à l'aide des dessins annexés, donnés à titre purement illustratif et non limitatif :
- La figure 1 illustre selon une vue en perspective de côté par l'avant un exemple d'implant vertébral selon l'invention composé de ses différents éléments.
- La figure 2 représente selon une vue en perspective partielle de côté par l'arrière, une cage intersomatique selon l'invention.
- La figure 3 illustre selon une vue en coupe longitudinale un exemple d'une vis de fixation selon l'invention.
- La figure 4 représente un exemple d'une vis de fixation vue de dessus dans un plan perpendiculaire à l'axe de rotation de la vis.
- La figure 5 représente selon une vue de côté un implant vertébral selon l'invention doté de vis de fixation vissées au travers de la cage.
- La figure 6 représente selon une vue en perspective frontale, l'implant vertébral selon l'invention de la figure 5 doté de vis de fixation vissées au travers de la cage.
- La figure 7 représente selon une vue en perspective par l'avant une variante d'implant vertébral selon l'invention, dotés de quatre vis de fixation vissées au travers de la cage.
- La figure 8 représente selon une vue en perspective par l'avant une variante de cage intersomatique dans pourvue d'un moyen de réglage de la hauteur de ladite cage selon l'invention.
- La figure 9A représente selon une vue de côté deux modules séparables destinés à être assemblés afin de former la cage intersomatique selon l'invention.
- La figure 9B représente selon une vue en perspective par l'avant les deux modules de la figure 9A assemblés formant la cage intersomatique selon l'invention.
- La figure 10 représente selon une vue en perspective un implant vertébral selon l'invention, comportant une plaque intersomatique dotée de deux vis de fixation.
- La figure 11 représente selon une vue en perspective une variante de la vis selon l'invention, laquelle comporte un filetage de tête.

### MEILLEURE MANIERE DE REALISER L'INVENTION

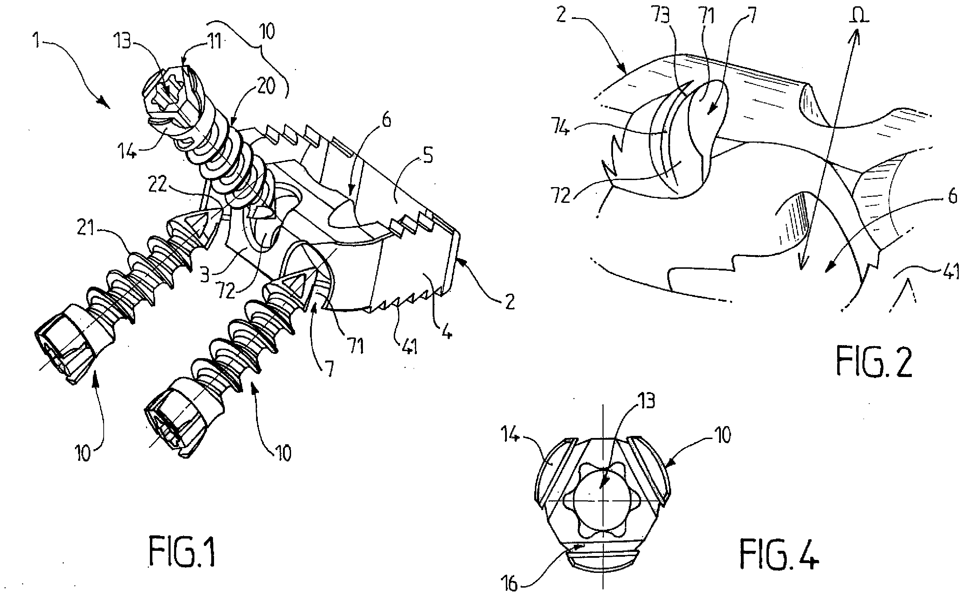

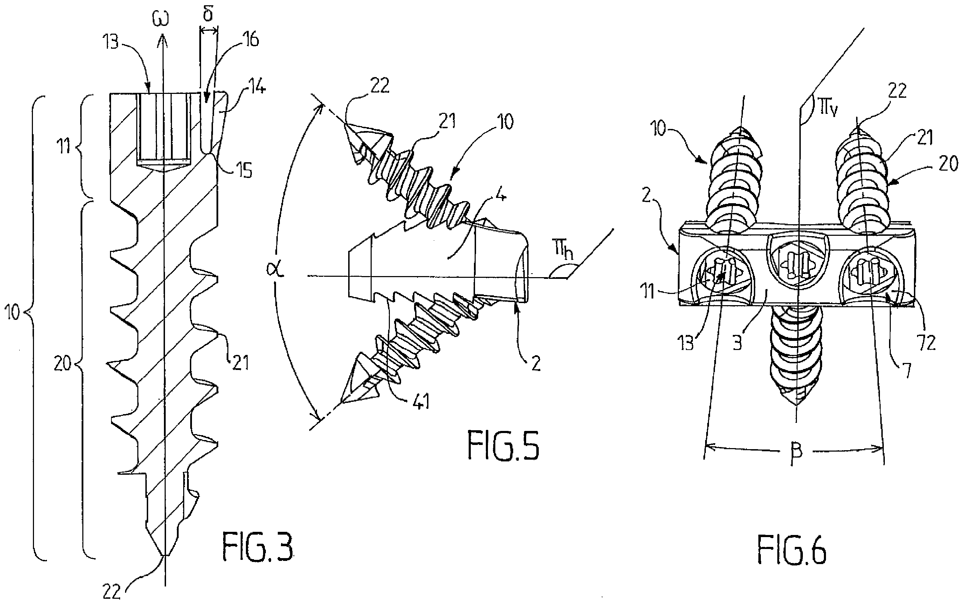

Un exemple d'implant vertébral 1 conforme à l'invention est illustré à la figure 1. Il comprend une cage intersomatique 2 et trois vis de fixation 10. Le nombre de vis ne correspond bien évidemment qu'à un exemple de réalisation et peut être inférieur ou supérieur à trois. Chaque vis 10 est composée d'une tête 11 de vis et d'un corps principal 20. La tête 11 s'étend du sommet de la vis 10 jusqu'au commencement d'un filet hélicoïdal 21 entourant le corps principal 20. Une empreinte 13 est creusée dans son épaisseur au sommet de la tête 11 de la vis 10. Elle permet de visser la vis 10 grâce à un objet de forme réciproque, de type tournevis. Une empreinte 13 de forme torx est représentée sur les différentes figures 1, 3, 4 et 6. Bien évidemment toute autre empreinte 13 de forme différente (phillips, hexagonale) peut être utilisée. La vis est ensuite prolongée par le corps principal 20 composé du filet hélicoïdal 21 qui est terminé par une pointe 22. Le filet 21 et la pointe 22 sont donc particulièrement adaptés pour un vissage au travers de la masse osseuse d'un patient. Les vis 10 définissent un axe de rotation ω qui est notamment représenté en trait plein sur la figure 3. La longueur de la vis 10, du sommet de la tête 11 à la pointe 22 est par exemple comprise entre 10 et 22 mm.

Avantageusement, la cage 2 est de forme sensiblement parallélépipédique assimilable à une arche. Ainsi, elle comprend une face avant 3, une face arrière 5 et deux bords latéraux 4. Les dimensions de la cage 2 vont notamment dépendre du lieu où elle est censée être implantée. En effet, l'implant 1 selon l'invention peut être destiné à être implanté dans l'espace intersomatique au niveau cervical ou lombaire.

Dans le cas d'une cage 2 cervicale, la distance entre les faces avant 3 et arrière 5 est comprise entre 11 et 16 mm, et la distance entre les deux bords latéraux est comprise entre 12 et 20 mm. La hauteur de la cage 2 est directement reliée à la pathologie du patient et est comprise entre 4,7 et 18 mm. Tandis que pour une cage lombaire, la distance entre les faces avant 3 et arrière 5 de la cage 2 est comprise entre 20 et 36 mm, et la distance entre les deux bords latéraux est comprise entre 10 et 36 mm. La hauteur de la cage 2 est quant à elle comprise entre 7 et 18 mm.

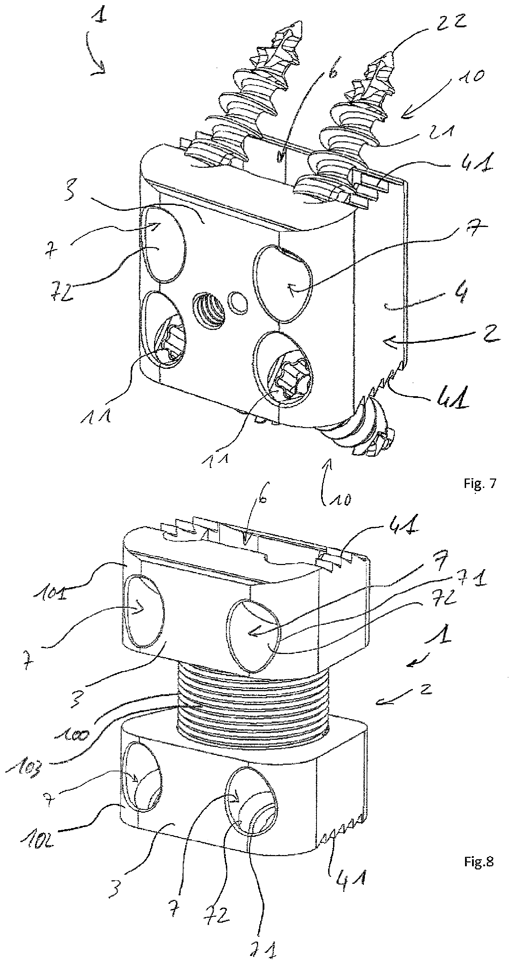

On choisit la hauteur de la cage 2 en fonction de la pathologie du patient et en fonction de l'espace intersomatique au sein duquel la cage 2 est destinée à se loger. Une variante de l'invention selon laquelle la cage 2 est de hauteur inférieure à la distance entre les faces avant 3 et arrière 5 est représentée à la figure 1. Une autre variante de l'invention selon laquelle la cage 2 est de hauteur sensiblement égale ou supérieure à la distance entre les faces avant 3 et arrière 5 est représentée à la figure 7.

La cage 2 comprend également un logement 6, au centre du parallélépipède, au sein duquel on peut déposer un matériel ostéinducteur avant d'introduire la cage 2 au sein de l'espace intersomatique d'un patient. Ce logement 6 ne comprend préférentiellement pas de plancher haut ni de plancher bas pour que le matériel ostéoformateur qu'il est censé recevoir puisse être en contact avec la masse osseuse du patient. Ainsi, la cage 2 est similaire à une arche, la face avant 3 correspondant au sommet de l'arche, les bords latéraux 4 aux piliers de l'arche et la face arrière 5 au plancher. Le logement 6 correspond ainsi avantageusement à l'ouverture d'une telle arche.

On peut dès lors aisément définir un plan médian vertical πᵥ de la cage 2, représenté schématiquement sur la figure 6, qui est le plan séparant la cage 2 en une partie gauche et une partie droite de dimension horizontale similaire. On peut également définir un plan médian horizontal πₕ de la cage 2, représenté schématiquement sur la figure 5, qui est le plan séparant la cage 2 en une partie supérieure et une partie inférieure de dimension verticale similaire.

Il est possible d'envisager une variante de l'invention, représentée à la figure 8, selon laquelle la cage 2 est de hauteur réglable. Pour cela, l'implant vertébral 1 comprend un moyen de réglage 100 de la hauteur de la cage 2.

De préférence, le moyen de réglage 100 est un élément d'élongation 103 disposé à proximité du plan médian horizontal πₕ de la cage 2 et séparant une partie supérieure 101 et une partie inférieure 102 de la cage 2, la mise en action de l'élément d'élongation 103 permettant d'éloigner et de tenir à la distance voulue la partie supérieure 101 de la partie inférieure 102 afin de faire varier la hauteur de ladite cage 2.

L'élément d'élongation 103 peut par exemple comprendre un système vis-écrou, ou un système de crémaillère. L'élément d'élongation 103 permet à la cage 2 d'adopter plusieurs tailles différentes afin d'être adaptable à plusieurs situations pathologiques chez le patient.

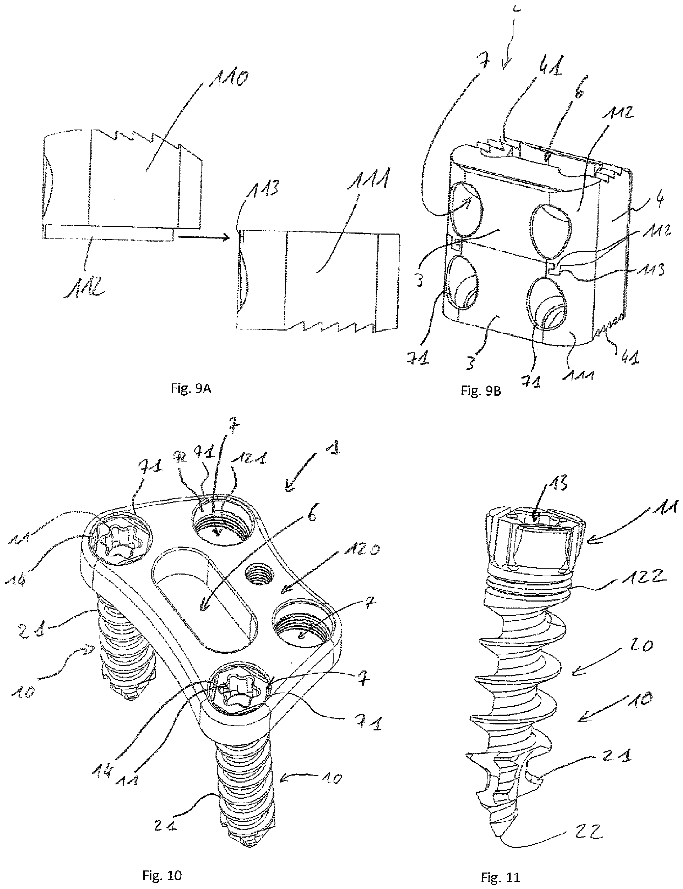

Il est possible d'envisager une autre variante de l'invention, représentée aux figures 9A et 9B, selon laquelle la cage 2 est modulaire, et comprend plusieurs modules séparables, de préférence au moins deux modules séparables, d'une part un module supérieur 110 comprenant un moyen mâle d'assemblage 112 et d'autre part un module inférieur 111 comprenant un moyen femelle d'assemblage 113, ledit moyen femelle d'assemblage 113 étant conçu pour coopérer avec le moyen mâle d'assemblage 112 afin d'assembler le module supérieur 110 avec le module inférieur 111 pour former ladite cage 2.

Dès lors, il est possible d'associer, en les assemblant, le module supérieur 110 avec le module inférieur 111, afin d'obtenir la cage 2 dont les propriétés telles que la forme, la hauteur, et / ou le nombre de puits 7, dépendront des propriétés combinées des modules supérieur 110 et inférieur 111 associés.

De manière préférentielle, le moyen mâle d'assemblage 112 comprend une glissière en L, le moyen femelle d'assemblage 113 comprenant une gorge en L de forme complémentaire à la glissière en L, la glissière et la gorge étant conçues pour coopérer à coulissement l'une avec l'autre, afin que l'assemblage du module inférieur 111 et du module supérieur 110 s'effectue par coulissement.

Dans l'objectif de stabiliser la position de la cage 2, la ou les vis 10 doivent la traverser pour l'arrimer solidement à la masse osseuse. Ainsi, la cage 2 comprend un ou des orifices 71 formant des puits 7 ménagés dans l'épaisseur de la cage 2.

Avantageusement, la face avant 3 de la cage 2, par exemple représentée au premier plan de la figure 6, est pourvue de tels orifices 71 formant des puits 7 dans l'épaisseur de la face avant 3. Préférentiellement, le puits 7 comporte un axe de révolution Ω avec des parois 72 symétriques en regard de cet axe. Cet axe de révolution Ω est notamment représenté sur l'orifice central de la cage 2 de la figure 2. Une fois la vis 10 positionnée dans la cage 2 au travers du puits 7, l'axe de révolution Ω du puits 7 et l'axe de rotation ω de la vis 10 sont confondus. Avantageusement, cet axe de révolution Ω forme un angle α compris entre 0 et 45 degrés, de manière plus préférentielle entre 20 et 40 degrés, avec le plan médian horizontal πₕ de la cage 2, comme cela est illustré sur la figure 5.

La face avant 3 est reliée à la face arrière 5 grâce aux bords latéraux 4 qui comprennent préférentiellement des dents rétentives 41 ménagées sur ses arêtes. Ces dents rétentives 41 vont permettre d'aider à la stabilisation de la cage au sein de l'espace intersomatique. Ces dents rétentives 41 sont notamment représentées de profil sur la figure 5. Elles sont en forme de dents de scie avec un angle compris entre 35 et 50 degrés et selon une hauteur verticale comprise entre 0,5 et 1,6 mm.

Enfin, la face arrière 5 est composée d'une plaque, par exemple rectangulaire, qui relie les deux bords latéraux 4 et solidifie l'ensemble de la structure de la cage.

Par ailleurs, les différents éléments de l'implant vertébral 1 selon l'invention sont réalisés dans des matériaux tels que le titane, le PEEK, le PEKK, les fibres de carbone ou en un matériau résorbable.

Avantageusement, la cage 2 selon l'invention est réalisée en usinage de titane et/ou en un matériau à base de titane pourvu de pores augmentant la surface d'échange entre la cage et l'extérieur, lesdits pores étant obtenus par la technique de fusion laser du titane. Cette technique permet de créer des matériaux comportant des pores pouvant communiquer entre eux. La précision du laser permet d'obtenir un contrôle précis de la macrostructure du matériau (taille des porosités, morphologie, distribution). L'augmentation de la surface d'échange entre la cage 2 et les tissus du patient permet d'améliorer sensiblement l'ostéosynthèse et donc l'efficacité de l'implant dans le traitement de la pathologie du patient.

L'implant 1 selon l'invention comprend au moins une vis de fixation 10 destinée à être vissée dans la masse osseuse au travers du puits 7 de la cage 2 pour stabiliser la position de la cage 2 au sein de l'espace intersomatique. Cette stabilisation est donc assurée par la vis 10 de fixation qui empêche tout mouvement de l'implant 1. Pour s'assurer du maintien en position de la cage 2 au cours du temps, il est primordial d'empêcher le dévissage de la vis 10 qui pourrait se produire suite aux micro-mouvements que peut générer le patient lors de déplacements quotidiens ou de mouvements particuliers.

A cet effet, le puits 7 et la vis 10 selon l'invention sont réalisés de manière à former un système de verrouillage autonome de la vis 10 dans le puits 7, ledit système comprenant un moyen mâle solidaire de la vis 10 et un moyen femelle formé par les parois 72 du puits 7. Lesdits moyens mâle et femelle sont montés avec une flexibilité relative autorisant lors du vissage de la vis 10 dans la masse osseuse au travers du puits 7, une mise en contrainte progressive et mutuelle des moyens mâle et femelle jusqu'à atteindre une position finale de verrouillage de la vis 10 dans laquelle les moyens mâle et femelle sont mutuellement relâchés, le moyen mâle étant verrouillé en position par le moyen femelle.

Ainsi, les parois 72 du puits 7 et le moyen mâle lié aux vis de fixation 10 entrent en contact lors du vissage de la vis 10 au travers du puits 7 de la cage 2. Il résulte de ce contact une contrainte appliquée sur le moyen mâle par le moyen femelle et réciproquement. Les formes géométriques respectives des moyens mâle et femelle créent une augmentation de cette contrainte, de préférence continue, lors de l'avancée de la vis 10 dans le puits 7.

De cette contrainte résulte une force qui va mettre en mouvement dans un certain sens respectif le moyen mâle et/ou le moyen femelle grâce à la flexibilité d'une ou plusieurs parties mobiles solidaires de l'un et/ou de l'autre moyen. Ce mouvement se poursuit jusqu'à ce que le moyen mâle atteigne la position finale où l'élasticité de la ou des parties mobiles permet au moyen mâle et/ou femelle d'effectuer un mouvement de direction opposée pour venir se placer dans une position relâchée.

La position finale est donc une position où le moyen mâle est verrouillé par le moyen femelle. Ce verrouillage s'est uniquement effectué par le vissage de la vis 10 dans le puits 7 de la cage 2. Dans cette position la vis 10 est solidaire de la cage 2, ce qui permet son maintien en position au cours du temps. Ce verrouillage empêche donc toute migration de l'implant.

De manière préférentielle, les moyens mâle et femelle sont montés avec une flexibilité relative autorisant lors du dévissage de la vis, lorsque les moyens mâle et femelle se trouvent dans ladite position finale, une mise en contrainte mutuelle des moyens mâle et femelle jusqu'à déverrouiller le moyen mâle du moyen femelle.

Ainsi, la flexibilité entre les moyens mâle et femelle qui a permis le verrouillage autonome est suffisante pour effectuer l'opération inverse suite à une action de dévissage. Les parties mobiles des moyens mâle et/ou femelle sont donc suffisamment élastiques pour permettre à la vis 10 de sortir de la position finale de verrouillage par simple action de dévissage. Les propriétés de flexibilité ainsi que la forme des moyens mâle et femelle sont finement choisies pour permettre cet aller-retour synonyme de verrouillage et de déverrouillage par respectivement des opérations de vissage et de dévissage.

Cette action de déverrouillage est donc uniquement mise en œuvre par une action de dévissage.

Avantageusement, ladite vis 10 comprend au moins une tête 11 et un corps principal 20, ledit moyen femelle est fixe et ledit moyen mâle est mobile et comporte un système escamotable de modification de la circonférence de ladite tête 11 permettant lors du vissage la flexibilité relative entre lesdits moyens mâle et femelle.

Lors du vissage, les parois 72 du puits 7 vont donc être en appui sur le système escamotable et modifier la circonférence de la tête 11 de la vis 10. Ce système escamotable constitue dès lors une partie mobile du moyen mâle.

De manière préférentielle, les parois 72 du puits 7 forment à partir de l'orifice 71 du puits 7 et selon la direction de vissage une première zone de diminution de la section du puits 7, ladite première zone permettant la compression radiale centripète dudit système escamotable, jusqu'à un épaulement 73 augmentant la section du puits et définissant une deuxième zone permettant l'expansion radiale centrifuge dudit système escamotable, ledit épaulement 73 formant également un lieu d'appui 74 circulaire, perpendiculaire à la direction de vissage, et destiné à être en contact avec ledit système escamotable lorsqu'il est en expansion radiale. Dans cette réalisation, le moyen femelle ne comporte pas de parties flexibles. La flexibilité est donc uniquement assurée par !e moyen escamotable.

Le rétrécissement du diamètre du puits 7 est de préférence continu. Le puits 7 a donc une forme conique à partir de son orifice 71 jusqu'à l'épaulement 73 comme cela est représenté notamment sur la figure 2. Les parois 72 du puits 7 vont donc appliquer une force d'amplitude grandissante sur le moyen escamotable. Cette force sera maximale à l'épaulement 73, juste avant d'atteindre la position finale.

L'épaulement 73 est le lieu où le diamètre du puits 7 passe de manière discontinue d'une valeur de diamètre minimum de l'épaulement 73 à une valeur de diamètre maximum de l'épaulement 73. Ainsi, le puits 7 comporte dès lors un lieu d'appui 74 formant une couronne autour de l'axe de révolution Ω du puits 7. La profondeur radiale de cette couronne est donc égale à la différence entre le diamètre maximum et le diamètre minimum de l'épaulement 73.

Cet épaulement 73 permet au moyen mâle de se relâcher en autorisant une augmentation de la circonférence de la tête 11 de la vis 10. Ainsi les moyens escamotables de la tête 11 lui permettent d'avoir un diamètre supérieur au diamètre minimum de l'épaulement 73. Avantageusement, la partie supérieure du moyen escamotable est en contact avec le lieu d'appui 74 ce qui permet de réaliser le verrouillage en position de la vis 10. L'écart de la valeur de la contrainte appliquée sur le moyen escamotable avant le passage de l'épaulement 73 et après constitue dès lors une barrière énergétique qui permet de caractériser cette position finale comme étant une position d'équilibre de la vis 10 au sein de la cage 2. La vis 10 ne peut pas être dans cette situation facilement délogée de la cage 2. Le maintien en position de la cage 2 est ainsi assuré.

En revanche, lors du dévissage de la vis 10 par un outil approprié, l'élasticité du moyen escamotable peut permettre à ce dernier de fléchir suffisamment pour permettre le passage de l'épaulement 73. Ce moyen escamotable est donc suffisamment flexible pour permettre son déverrouillage par une simple action de dévissage mais suffisamment rigide pour permettre son maintien en position lorsqu'il ne subit que les micro-mouvements induits par les déplacements du patient.

Selon la réalisation préférentielle de l'invention, ce système escamotable peut être formé par au moins une languette 14 longitudinale, jointive par sa base 15 de ladite tête 11 et formant ainsi au moins un pétale s'étendant radialement autour de ladite tête 11, ladite au moins une languette 14 étant montée mobile élastiquement et présentant une position de repos élastique. Les figures 1, 3 et 4 permettent d'illustrer clairement cette réalisation préférentielle de l'invention.

La figure 3 représente selon une vue en coupe une vis 10 dont la tête 11 est pourvue d'une languette 14 qui est rattachée à la vis 10 uniquement au niveau de sa base 15. Cette jonction permet à la languette 14 de pouvoir se déplacer dans une direction radiale (perpendiculaire à l'axe de rotation ω de la vis 10), ce qui a pour effet de modifier la circonférence de la tête 11 de la vis 10. Cette languette 14 est également élastique car elle se place dans une position naturelle de repos élastique lorsqu'elle ne subit aucune contrainte.

Avantageusement, dans la position de repos élastique, la languette 14 longitudinale est écartée de l'axe de rotation ω de la vis 10 d'un angle δ compris entre 0 et 25 degrés, préférentiellement entre 0 et 9 degrés, comme cela est notamment représenté sur la figure 3. Ainsi, cette languette 14 peut être écartée angulairement de l'axe de rotation ω de la vis 10 ou être sensiblement parallèle à cet axe ω. Plus cet angle δ sera élevé, plus la différence d'éloignement au centre de la vis 10 entre la base 15 et le sommet de la languette 14 sera importante. Si cette languette 14 est sensiblement parallèle à l'axe de rotation ω de la vis 10, l'éloignement au centre de la vis 10 sera constant en tout point de languette 14 dans la position de repos élastique.

L'ajustement de cet angle δ de séparation entre la languette14 et l'axe de rotation ω de la vis 10 permet notamment d'ajuster les propriétés d'élasticité de la languette 14 mobile pour lui permettre de verrouiller et de déverrouiller la vis 10 et le puits 7.

Il existe par construction un espace vide 16 qui sépare la languette 14 de la tête 11 de la vis 10. Ainsi, lorsqu'une languette 14 est mise sous contrainte par les parois du puits, elle se rapproche de l'axe de rotation de la vis 10 grâce à l'élasticité de cette partie mobile et de la présence de l'espace vide 16. Plus cet espace 16 sera grand, plus la vis pourra potentiellement se déplacer avec une amplitude importante. Bien évidemment d'autres paramètres influent sur l'amplitude de ce déplacement, comme la hauteur, selon l'axe de rotation ω de la vis 10, de la languette 14 ou l'angle δ de séparation défini précédemment.

Avantageusement, le bord externe de la languette 14 est de forme arrondie pour permettre une mise en contrainte aisée de la vis par les parois 72 du puits 7 lors de la rotation induite par le vissage.

De manière préférentielle, la languette 14 est réalisée dans un matériau lui assurant des propriétés de souplesse pour pouvoir être suffisamment mobile sous contrainte tout en gardant une certaine rigidité pour assurer la qualité du verrouillage.

Avantageusement, ladite vis 10 de fixation comporte trois languettes 14 longitudinales disposées autour de sa tête 11, comme cela est notamment représenté sur les figures 1, 3 et 4.

La figure 4 représente notamment une vis 10 selon l'invention sur laquelle trois languettes 14 sont équiréparties angulairement autour de la tête 11 de la vis 10. Chaque languette 14 s'étend angulairement autour de la vis 10 d'un angle compris entre 60 et 120 degrés. Le nombre de ces languettes 14 est ajusté pour permettre une bonne stabilisation de la vis 10 lorsqu'elle est verrouillée dans ladite position finale. Une vis 10 comportant plus ou moins de languettes 14 aura bien évidemment un principe de fonctionnement similaire.

Les vis 10 sont vissées dans la masse osseuse du patient en traversant la cage 2. Les directions de vissage sont déterminées dans l'objectif de mieux stabiliser la position de la cage 2 au sein de l'espace intersomatique.

Avantageusement l'implant vertébral 1 selon l'invention comporte plusieurs vis de fixation 10 et plusieurs puits 7, de préférence trois vis et trois puits. En effet, le nombre de vis 10 influe directement sur la tenue de la cage 2 au sein de l'espace intersomatique en augmentant les points de fixation.

De manière préférentielle, l'axe de révolution Ω des puits 7 forme un angle α compris entre 0 et 45 degrés, préférentiellement entre 20 et 40 degrés, avec le plan médian horizontal πₕ de ladite cage, comme cela est notamment représenté sur la figure 5.

Cela permet de clairement définir l'angle de pénétration de la vis 10 dans la masse osseuse du patient. Dans cette configuration, les vis 10 prennent appui sur la masse osseuse située au-dessus et au-dessous de l'implant 1. Cette multitude de points de fixation aide à la stabilisation de la cage 2.

Préférentiellement, les orifices 71 des puits 7 sont ménagés sur la face avant 3 de la cage 2 pour permettre au praticien d'introduire facilement puis visser les vis 10 dans la masse osseuse du patient car la position des orifices est ainsi facilement accessible. Lesdits puits 7 sont façonnés pour que la majeure partie desdites vis 10 soient placées alternativement au-dessus et au-dessous du plan médian horizontal πₕ de ladite cage 2.

Avantageusement, l'axe de révolution Ω des puits 7 forme un angle β avec le plan médian vertical de ladite cage compris entre 0 et 20 degrés, comme cela est notamment représenté sur la figure 6. Les vis 10 traversant ces puits 7 sont orientées telles que les pointes 22 soient plus proches du plan médian vertical πᵥ que les têtes 11. Cela permet de s'assurer que les vis 10 pénètrent bien la masse osseuse.

Selon une variante de l'invention illustrée à la figure 7, l'implant vertébral 1 comporte de préférence quatre vis de fixation 10 et quatre puits 7.

En effet, augmenter le nombre de vis 10 peut permettre d'augmenter la tenue de la cage 2 au sein de l'espace intersomatique en augmentant le nombre de points de fixation, notamment dans le cas où la cage 2 est de taille suffisante pour accueillir quatre puits 7 et quatre vis de fixation 10.

De préférence, les quatre vis de fixation 10 et les quatre puits 7 sont placés sur la cage 2 et orientés de manière symétrique deux à deux.

Un nombre de vis de fixation 10 pair permet de répartir les points de fixation de manière symétrique, les vis de fixation 10 pouvant être disposées et orientées deux à deux, avec par exemple des angles de pénétration respectifs complémentaires, afin d'améliorer la tenue de la cage 2 au sein de l'espace intersomatique.

A titre de variante, le moyen mâle étant formé par la tête 11 de vis 10, les parois 72 du puits 7 comportent des portions mobiles permettant une modification de la circonférence du puits 7, autorisant lors du vissage ladite flexibilité relative entre lesdits moyens mâle et femelle. Une telle variante n'est pas représentée sur les différentes figures.

Dans une telle situation le vissage de la vis 10 au travers du puits 7 va permettre le déplacement des parties mobiles des parois 72 du puits 7. Le passage de la vis 10 va ensuite permettre le relâchement de ces parois mobiles et donc le verrouillage de la vis 10 au sein de la cage 2. Cet exemple de réalisation non préférentielle ne sera pas détaillé mais illustre bien toutes les variantes possibles de la présente invention.

L'implant vertébral 1 selon une variante de l'invention représentée à la figure 1, est destiné à être implanté de préférence entre deux os de la colonne vertébrale du patient, de préférence au niveau cervical ou lombaire. La forme de la cage 2 est particulièrement adaptée à une telle implantation.

L'invention concerne également un implant vertébral 1 destiné à être implanté le long de l'espace intersomatique d'un patient et comprenant une plaque intersomatique 120 et au moins une vis de fixation 10 destinée à être vissée dans la masse osseuse au travers d'un puits 7 de ladite plaque 120 pour stabiliser la position de ladite plaque 120 le long de l'espace intersomatique, ledit implant 1 étant caractérisé en ce que le puits 7 et la vis 10 forment un système de verrouillage autonome de la vis 10 dans le puits 7, ledit système comprenant un moyen mâle solidaire de la vis 10 et un moyen femelle formé par les parois 72 du puits 7, lesdits moyens mâle et femelle étant montés avec une flexibilité relative autorisant lors du vissage de la vis 10 dans la masse osseuse au travers du puits 7, une mise en contrainte progressive et mutuelle des moyens mâle et femelle jusqu'à atteindre une position finale de verrouillage de la vis 10 dans laquelle les moyens mâle et femelle sont mutuellement relâchés, le moyen mâle étant verrouillé en position par le moyen femelle, ladite vis 10 comprenant au moins une tête 11 et un corps principal 20, ledit moyen femelle étant fixe et ledit moyen mâle étant mobile et comportant un système escamotable de modification de la circonférence de ladite tête 11 permettant lors du vissage la flexibilité relative entre lesdits moyens mâle et femelle.

La plaque intersomatique 120 est particulièrement adaptée pour que l'implant vertébral 1 puisse être implanté dans la masse osseuse de la colonne vertébrale d'un patient par voie latérale, par voie postérieure, ou par voie antérieure. La plaque intersomatique 120 est par exemple une plaque cervicale, ou de préférence une plaque thoraco-lombaire.

De préférence, les moyens mâle et femelle sont montés avec une flexibilité relative autorisant lors du dévissage de la vis 10, à partir de ladite position finale, une mise en contrainte mutuelle des moyens mâle et femelle jusqu'à déverrouiller le moyen mâle du moyen femelle.

De manière préférentielle, les parois 72 du puits 7 forment à partir de l'orifice 71 du puits 7 et selon la direction de vissage une première zone de diminution de la section du puits 7, ladite première zone permettant la compression radiale centripète dudit système escamotable, jusqu'à un épaulement 73 augmentant la section du puits 7 et définissant une deuxième zone permettant l'expansion radiale centrifuge dudit système escamotable, ledit épaulement 73 formant également un lieu d'appui circulaire 74, perpendiculaire à la direction de vissage, et destiné à être en contact avec ledit système escamotable lorsqu'il est en expansion radiale.

Préférentiellement, le système escamotable est formé par au moins une languette longitudinale 14, jointive par sa base 15 de ladite tête 11 et formant ainsi au moins un pétale s'étendant radialement autour de ladite tête 11, ladite au moins une languette 14 étant montée mobile élastiquement et présentant une position de repos élastique.

De préférence, la vis 10 comporte trois languettes longitudinales 14 disposées autour de sa tête 11.

Préférentiellement, la position de repos élastique, ladite languette longitudinale 14 est écartée de l'axe de rotation ω de la vis 10 d'un angle δ compris entre 0 et 25 degrés, préférentiellement entre 0 et 9 degrés.

De façon préférentielle, l'implant vertébral 1 comporte plusieurs vis 10 et plusieurs puits 7, de préférence trois vis 10 et trois puits 7.

Préférentiellement en outre, la vis 10 comprend au moins une tête 11 et un corps principal 20, ledit moyen mâle étant formé par ladite tête 11 et les parois 72 du puits 7 comportant des portions mobiles permettant une modification de la circonférence du puits 7, autorisant lors du vissage ladite flexibilité relative entre lesdits moyens mâle et femelle.

Selon la variante représentée aux figures 10 et 11, le puits 7 comporte un filetage interne 121 destiné à coopérer avec un filetage de tête 122 de la vis, ledit filetage de tête 122 étant situé au niveau de la tête de la vis 11.

Lorsque la vis est introduite dans le puits 7, l'interaction du filetage interne 121 avec le filetage de tête 122 contribue au verrouillage de ladite vis 10 dans le puits 7.

Ainsi, une même vis de fixation 10 peut être associée soit à une cage intersomatique 2, soit à une plaque intersomatique 120, en vue de former un implant vertébral 1 s'adaptant aux différentes situations pathologiques du patient.

De ce fait, l'invention concerne également un implant vertébral 1 comprenant une cage intersomatique 2 destinée à être implanté dans l'espace intersomatique d'un patient ou comprenant une plaque intersomatique 120 destinée à être implantée le long de l'espace intersomatique d'un patient et au moins une vis de fixation 10 destinée à être vissée dans la masse osseuse au travers d'un puits 7 de ladite cage 2 ou de ladite plaque 120 pour stabiliser la position de ladite cage 2 au sein de l'espace intersomatique ou de ladite plaque 120 le long de l'espace intersomatique, ledit implant 1 étant caractérisé en ce que le puits 7 et la vis 10 forment un système de verrouillage autonome de la vis 10 dans le puits 7, ledit système comprenant un moyen mâle solidaire de la vis 10 et un moyen femelle formé par les parois 72 du puits 7, lesdits moyens mâle et femelle étant montés avec une flexibilité relative autorisant lors du vissage de la vis 10 dans la masse osseuse au travers du puits 7, une mise en contrainte progressive et mutuelle des moyens mâle et femelle jusqu'à atteindre une position finale de verrouillage de la vis 10 dans laquelle les moyens mâle et femelle sont mutuellement relâchés, le moyen mâle étant verrouillé en position par le moyen femelle, ladite vis 10 comprenant au moins une tête 11 et un corps principal 20, ledit moyen femelle étant fixe et ledit moyen mâle étant mobile et comportant un système escamotable de modification de la circonférence de ladite tête 11 permettant lors du vissage la flexibilité relative entre lesdits moyens mâle et femelle.

### METHODE DE MISE EN PLACE DE L'IMPLANT 1

Les différentes étapes permettant de placer un implant vertébral 1 comprenant une cage intersomatique 2 et au moins une vis de fixation 10, dans l'espace intersomatique d'un patient, au niveau cervical ou lombaire, sont décrites ci-après.

L'implant vertébral 1 est destiné à être implanté dans l'espace intersomatique d'un patient par voie antérieure ou postérieure.

Préférentiellement, on peut disposer dans le logement 6 de la cage 2 un matériel ostéoinducteur, tel qu'un greffon osseux et/ ou un substitut, qui permettra la reconstruction osseuse.

Pour introduire la cage 2 dans l'espace intersomatique du patient, un ancillaire spécifique est utilisé. Avantageusement, ladite cage 2 comporte une ou plusieurs encoches conjuguées aux formes de l'ancillaire afin d'assurer une préhension de la cage. Pour que cette tenue entre l'ancillaire et la cage 2 soit efficace et empêche notamment tout lâcher intempestif, la cage 2 comporte une ou plusieurs zones de préhension (non représentées). Elles sont situées de préférence sur les faces latérales de la cage 2 et sont constituées d'une ou plusieurs encoches de forme jumelle à des contre-encoches présentes sur l'ancillaire adapté. De manière préférentielle, ces encoches sont asymétriques sur chaque bord latéral 4 de la cage 2 pour s'assurer que la cage 2 soit correctement implantée par le praticien au sein de l'espace intersomatique en n'autorisant qu'un sens de préhension. Cela permet d'éviter une pose inversée de l'implant 1 et constitue ainsi un système de détrompage.

L'invention concerne également dès lors un kit médical formé par un implant vertébral 1 conforme à l'invention et un ancillaire de mise en place dudit implant vertébral 1 dans l'espace intersomatique d'un patient.

Après avoir saisi la cage 2 grâce à l'ancillaire spécifique, la cage 2 est placée dans l'espace intersomatique du patient, La zone de dépôt de la cage 2 a été ménagée préalablement par le praticien pour permettre à cette zone d'accueillir l'implant 1.

Puis, on visse au moins une vis de fixation 10 au travers d'un orifice 71 de ladite cage dans la masse osseuse du patient, l'étape de vissage assurant le vissage de la vis 10 dans la masse osseuse puis le verrouillage dans la position finale de la vis 10 au sein de la cage 2.

Dans la réalisation préférentielle de l'invention, lorsque la tête 11 de la vis 10 atteint le puits 7 au cours de son vissage, les parois 72 vont notamment appuyer sur les languettes longitudinales 14 qui sont montés élastiquement mobiles. Lors de l'avancée de la vis 10, les languettes longitudinales 14 vont fléchir et se rapprocher du centre de la vis 10 et donc diminuer le diamètre de la tête 11 de la vis 10 jusqu'à atteindre l'épaulement 73. A ce niveau, l'avancée de la vis 10 a pour conséquence une expansion radiale des languettes 14 et donc une augmentation du diamètre de la tête 11 de la vis 10. Le lieu d'appui 74 formé par l'épaulement 73 empêche un dévissage de la vis 10 placée dans cette position finale de verrouillage. Cela permet d'assurer ainsi la stabilité de la position de la cage 2 au sein de l'espace intersomatique.

On peut également retirer l'implant 1 de l'espace intersomatique d'un patient si cela s'avère nécessaire. Pour cela, on retire la ou les vis de fixation 10 placées dans ladite position finale de la masse osseuse du patient par une action de dévissage, ladite action de dévissage débutant par le déverrouillage des vis 10.

Selon la réalisation préférentielle de l'invention, l'action de dévissage va créer une force sur les languettes 14 en prenant notamment appui sur la masse osseuse du patient par l'intermédiaire des filets 21 de la vis 10. Cela va générer une force suffisante, et supérieure aux forces subies habituellement, sur les languettes 14 pour utiliser leur élasticité et les déplacer vers le centre de la vis 10 afin que le diamètre de la tête 11 de la vis 10 devienne inférieur au diamètre minimum de l'épaulement 73. Une fois cet épaulement 73 franchi, la vis 10 est facilement retirée en continuant l'action de dévissage.

Une fois l'ensemble des vis 10 enlevé, on retire la cage 2 de l'espace intersomatique du patient.

Par ailleurs, la plaque intersomatique 120 est particulièrement adaptée pour que l'implant vertébral 1 puisse être implanté dans la masse osseuse de la colonne vertébrale d'un patient par voie latérale, par voie postérieure, ou par voie antérieure.

Ainsi, l'implant vertébral 1 selon l'invention propose une solution peu invasive pour s'assurer du maintien en position au cours du temps d'une cage 2 au sein de l'espace intersomatique d'un patient.

L'implant vertébral 1 selon l'invention offre également la possibilité d'être aisément retiré du corps du patient si cela s'avère nécessaire.

### POSSIBILITE D'APPLICATION INDUSTRIELLE

L'invention trouve son application industrielle dans la conception et la fabrication d'implants vertébraux et de leur système de fixation dans les os d'un patient.

## Revendications

1. Implant vertébral (1) destiné à être implanté dans l'espace intersomatique d'un patient et comprenant une cage intersomatique (2) et au moins une vis de fixation (10) destinée à être vissée dans la masse osseuse au travers d'un puits (7) de ladite cage (2) pour stabiliser la position de ladite cage (2) au sein de l'espace intersomatique, ledit puits (7) et la vis (10) formant un système de verrouillage autonome de la vis (10) dans le puits (7), ledit système comprenant un moyen mâle solidaire de la vis (10) et un moyen femelle formé par les parois (72) du puits (7), lesdits moyens mâle et femelle étant montés avec une flexibilité relative autorisant lors du vissage de la vis (10) dans la masse osseuse au travers du puits (7), une mise en contrainte progressive et mutuelle des moyens mâle et femelle jusqu'à atteindre une position finale de verrouillage de la vis (10) dans laquelle les moyens mâle et femelle sont mutuellement relâchés, le moyen mâle étant verrouillé en position par le moyen femelle, ladite vis (10) comprenant au moins une tête (11) et un corps principal (20), ledit moyen femelle étant fixe et ledit moyen mâle étant mobile et comportant un système escamotable de modification de la circonférence de ladite tête (11) permettant lors du vissage la flexibilité relative entre lesdits moyens mâle et femelle, lesdits moyens mâle et femelle étant montés avec une flexibilité relative autorisant lors du dévissage de la vis (10), à partir de ladite position finale, une mise en contrainte mutuelle des moyens mâle et femelle jusqu'à déverrouiller le moyen mâle du moyen femelle.

2. Implant vertébral (1) selon l'une des revendications précédentes, **caractérisé en ce que** les parois (72) du puits (7) forment à partir de l'orifice (71) du puits (7) et selon la direction de vissage une première zone de diminution de la section du puits (7), ladite première zone permettant la compression radiale centripète dudit système escamotable, jusqu'à un épaulement (73) augmentant la section du puits (7) et définissant une deuxième zone permettant l'expansion radiale centrifuge dudit système escamotable, ledit épaulement (73) formant également un lieu d'appui circulaire (74), perpendiculaire à la direction de vissage, et destiné à être en contact avec ledit système escamotable lorsqu'il est en expansion radiale.

3. Implant vertébral (1) selon l'une des revendications précédentes, **caractérisé en ce que** ledit système escamotable est formé par au moins une languette longitudinale (14), jointive par sa base (15) de ladite tête (11) et formant ainsi au moins un pétale s'étendant radialement autour de ladite tête (11), ladite au moins une languette (14) étant montée mobile élastiquement et présentant une position de repos élastique.

4. Implant vertébral (1) selon la revendication précédente **caractérisé en ce que** ladite vis (10) comporte trois languettes longitudinales (14) disposées autour de sa tête (11).

5. Implant vertébral (1) selon l'une des deux précédentes revendications **caractérisé en ce que**, dans la position de repos élastique, ladite languette longitudinale (14) est écartée de l'axe de rotation (ω) de la vis (10) d'un angle (δ) compris entre 0 et 25 degrés, préférentiellement entre 0 et 9 degrés.

6. Implant vertébral (1) selon l'une des revendications précédentes **caractérisé en ce que** ledit puits (7) a un axe de révolution (Ω) qui forme un angle (α) compris entre 0 et 45 degrés, préférentiellement entre 20 et 40 degrés, avec le plan médian horizontal (πₕ) de ladite cage.

7. Implant vertébral (1) selon l'une des revendications précédentes **caractérisé en ce qu'**il comporte plusieurs vis (10) et plusieurs puits (7), de préférence trois vis (10) et trois puits (7).

8. Implant vertébral (1) selon l'une des revendications précédentes **caractérisé en ce que** ladite cage (2) comporte une ou plusieurs encoches conjuguées aux formes d'un ancillaire spécifique afin d'assurer une préhension de la cage.

9. Implant vertébral (1) selon la revendication 1 **caractérisé en ce que** ladite vis (10) comprend au moins une tête (11) et un corps principal (20), ledit moyen mâle étant formé par ladite tête (11) et les parois (72) du puits (7) comportant des portions mobiles permettant une modification de la circonférence du puits (7) autorisant lors du vissage ladite flexibilité relative entre lesdits moyens mâle et femelle.

10. Implant vertébral (1) selon l'une des revendications précédentes **caractérisé en ce que** ses différents éléments sont réalisés dans des matériaux tels que le titane, le PEEK, le PEKK, les fibres de carbone ou en un matériau résorbable.

11. Implant vertébral (1) selon l'une des revendications précédentes **caractérisé en ce que** ladite cage (2) est réalisée en un matériau à base de titane pourvu de pores augmentant la surface d'échange entre la cage (2) et l'extérieur, lesdits pores étant obtenus par la technique de fusion laser du titane.

12. Implant vertébral (1) selon l'une des revendications précédentes **caractérisé en ce qu'**il comprend un moyen de réglage (100) de la hauteur de la cage (2).

13. Implant vertébral (1) selon la revendication précédente, **caractérisé en ce que** ledit moyen de réglage (100) comporte un élément d'élongation (103) disposé à proximité d'un plan médian horizontal (πₕ) de la cage (2) et séparant une partie supérieure (101) et une partie inférieure (102) de la cage (2), la mise en action de l'élément d'élongation (103) permettant d'éloigner et de tenir à la distance voulue la partie supérieure (101) de la partie inférieure (102) afin de faire varier la hauteur de ladite cage (2).

14. Implant vertébral (1) selon l'une des revendications précédentes, **caractérisé en ce que** ladite cage (2) est modulaire et comprend plusieurs modules séparables.

15. Implant vertébral (1) selon la revendication précédente, **caractérisé en ce que** ladite cage (2) comprend au moins deux modules séparables, d'une part un module supérieur (110) comprenant un moyen mâle d'assemblage (112) et d'autre part un module inférieur (111) comprenant un moyen femelle d'assemblage (113), ledit moyen femelle d'assemblage (113) étant conçu pour coopérer avec le moyen mâle d'assemblage (112) afin d'assembler le module supérieur (110) avec le module inférieur (111) pour former ladite cage (2).

16. Implant vertébral (1) selon la revendication précédente, **caractérisé en ce que** le moyen mâle d'assemblage (112) comprend une glissière en L, le moyen femelle d'assemblage (113) comprenant une gorge en L de forme complémentaire à la glissière en L, la glissière (112) et la gorge (113) étant conçues pour coopérer à coulissement l'une avec l'autre, afin que l'assemblage du module inférieur (111) et du module supérieur (110) s'effectue par coulissement.

17. Implant vertébral (1) selon l'une des revendications précédentes, **caractérisé en ce qu'**il est destiné à être implanté de préférence entre deux os de la colonne vertébrale du patient, de préférence au niveau cervical ou lombaire.

18. Implant vertébral (1) destiné à être implanté le long de l'espace intersomatique d'un patient et comprenant une plaque intersomatique (120) et au moins une vis de fixation (10) destinée à être vissée dans la masse osseuse au travers d'un puits (7) de ladite plaque (120) pour stabiliser la position de ladite plaque (120) le long de l'espace intersomatique, le puits (7) et la vis (10) formant un système de verrouillage autonome de la vis (10) dans le puits (7), ledit système comprenant un moyen mâle solidaire de la vis (10) et un moyen femelle formé par les parois (72) du puits (7), lesdits moyens mâle et femelle étant montés avec une flexibilité relative autorisant lors du vissage de la vis (10) dans la masse osseuse au travers du puits (7), une mise en contrainte progressive et mutuelle des moyens mâle et femelle jusqu'à atteindre une position finale de verrouillage de la vis (10) dans laquelle les moyens mâle et femelle sont mutuellement relâchés, le moyen mâle étant verrouillé en position par le moyen femelle, ladite vis (10) comprenant au moins une tête (11) et un corps principal (20), ledit moyen femelle étant fixe et ledit moyen mâle étant mobile et comportant un système escamotable de modification de la circonférence de ladite tête (11) permettant lors du vissage la flexibilité relative entre lesdits moyens mâle et femelle, lesdits moyens mâle et femelle étant montés avec une flexibilité relative autorisant lors du dévissage de la vis (10), à partir de ladite position finale, une mise en contrainte mutuelle des moyens mâle et femelle jusqu'à déverrouiller le moyen mâle du moyen femelle.

19. Implant vertébral (1) selon la revendication 18, **caractérisé en ce que** les parois (72) du puits (7) forment à partir de l'orifice (71) du puits (7) et selon la direction de vissage une première zone de diminution de la section du puits (7), ladite première zone permettant la compression radiale centripète dudit système escamotable, jusqu'à un épaulement (73) augmentant la section du puits (7) et définissant une deuxième zone permettant l'expansion radiale centrifuge dudit système escamotable, ledit épaulement (73) formant également un lieu d'appui circulaire (74), perpendiculaire à la direction de vissage, et destiné à être en contact avec ledit système escamotable lorsqu'il est en expansion radiale.

20. Implant vertébral (1) l'une des revendications 18 à 19, **caractérisé en ce que** ledit système escamotable est formé par au moins une languette longitudinale (14), jointive par sa base (15) de ladite tête (11) et formant ainsi au moins un pétale s'étendant radialement autour de ladite tête (11), ladite au moins une languette (14) étant montée mobile élastiquement et présentant une position de repos élastique.

21. Implant vertébral (1) selon la revendication précédente **caractérisé en ce que** ladite vis (10) comporte trois languettes longitudinales (14) disposées autour de sa tête (11).

22. Implant vertébral (1) selon l'une des deux précédentes revendications **caractérisé en ce que**, dans la position de repos élastique, ladite languette longitudinale (14) est écartée de l'axe de rotation (ω) de la vis (10) d'un angle (δ) compris entre 0 et 25 degrés, préférentiellement entre 0 et 9 degrés.

23. Implant vertébral (1) selon l'une des revendications 18 à 22 **caractérisé en ce qu'**il comporte plusieurs vis (10) et plusieurs puits (7), de préférence trois vis (10) et trois puits (7).

24. Implant vertébral (1) selon la revendication 18 **caractérisé en ce que** ladite vis (10) comprend au moins une tête (11) et un corps principal (20), ledit moyen mâle étant formé par ladite tête (11) et les parois (72) du puits (7) comportant des portions mobiles permettant une modification de la circonférence du puits (7), autorisant lors du vissage ladite flexibilité relative entre lesdits moyens mâle et femelle.

25. Implant vertébral (1) selon l'une des revendications 18 à 24 **caractérisé en ce que** le puits (7) comporte un filetage interne (121) destiné à coopérer avec un filetage de tête (122) de la vis, ledit filetage de tête (122) étant situé au niveau de la tête de la vis (11).

26. Kit médical formé par un implant vertébral (1) conforme à l'une des revendications précédentes et un ancillaire de mise en place dudit implant vertébral dans ou le long de l'espace intersomatique d'un patient.

## Patentansprüche

1. Wirbelsäulenimplantat (1), das in einen intersomatischen Raum eines Patienten implantiert werden soll und einen Zwischenwirbelkäfig (2) und mindestens eine Fixierschraube (10) umfasst, die durch eine Bohrung (7) des Käfigs (2) in die Knochenmasse geschraubt werden soll, um die Position des Käfigs (2) in dem intersomatischen Raum zu stabilisieren, worin die Bohrung (7) und die Schraube (10) ein System zum Selbstblockieren der Schraube (10) in der Bohrung (7) ausbilden, worin das System einen mit der Schraube (10) einstückigen männlichen Blockierbereich und einen durch die Wände (72) der Bohrung (7) ausgebildeten weiblichen Blockbereich umfasst, worin der männliche Blockierbereich und der weibliche Blockbereich mit einer relativen Elastizität befestigt sind, die während des Einschraubens der Schraube (10) in die Knochenmasse durch die Bohrung (7), ein fortlaufendes und gegenseitiges Belasten des männlichen Blockierbereiches und des weiblichen Blockbereiches ermöglichen, bis eine finale Blockierposition der Schraube (10) erreicht ist, in welcher der männliche Blockierbereich und der weibliche Blockbereich gegenseitig entlastet sind, worin der männliche Blockierbereich durch den weiblichen Blockbereich in Position gesichert ist, worin die Schraube (10) mindestens einen Kopf (11) und einen Hauptkörper (20) umfasst, wobei der weibliche Blockbereich befestigt ist und der männliche Blockierbereich mobil ist und ein zurückziehbares System zum Anpassen des Umfanges des Kopfes (11) umfasst, das während des Schraubens die relative Elastizität zwischen dem männlichen Blockierbereich und dem weiblichen Blockbereich ermöglicht, wobei der männliche Blockierbereich und der weibliche Blockbereich mit einer relativen Elastizität befestigt sind, die während des Abschraubens der Schraube (10) aus der finalen Position ein gegenseitiges Belasten des männlichen Blockierbereiches und des weiblichen Blockbereiches ermöglicht, bis der männliche Blockierbereich von dem weiblichen Blockbereich freigegeben ist.

2. Wirbelsäulenimplantat (1) nach dem vorstehenden Anspruch, **dadurch gekennzeichnet, dass** die Wände (72) der Bohrung (7), von einer Öffnung (71) der Bohrung (7) und in die Schraubrichtung eine erste Fläche der Verringerung des Bohrungsquerschnittes (7) bis zu einer Schulter (73) ausbilden, bis zu welcher der Bohrungsquerschnitt (7) ansteigt und eine zweite Fläche definiert, worin die erste Fläche die radiale zentripetale Kompression des zurückziehbaren Systems ermöglicht, worin die zweite Fläche die radiale zentrifugale Ausdehnung des zurückziehbaren Systems ermöglich, worin die Schulter (73) zudem einen runden Lagerort (74) ausbildet, der senkrecht zu der Schraubrichtung, und ausgestaltet ist, mit dem zurückziehbaren System in Kontakt zu kommen, wenn die Schulter (73) in radialer Ausdehnung ist.

3. Wirbelsäulenimplantat (1) nach einem der vorstehenden Ansprüche, **dadurch gekennzeichnet, dass** das zurückziehbare System durch mindestens einen Längsstreifen (14) ausgebildet wird, der mit seiner Basis (15) mit dem Kopf (11) verbunden ist und daher mindestens ein sich radial über den Kopf (11) erstreckendes Blatt ausbildet, worin der mindestens eine Streifen (14) elastisch mobil befestigt ist und eine elastische Ruheposition aufweist.

4. Wirbelsäulenimplantat (1) nach einem der vorstehenden Ansprüche **dadurch gekennzeichnet, dass** die Schraube (10) drei Längsstreifen (14) umfasst, die über dem Kopf (11) angeordnet sind.

5. Wirbelsäulenimplantat (1) nach einem der vorstehenden Ansprüche **dadurch gekennzeichnet, dass** in der elastischen Ruheposition der Längsstreifen (14) von einer Drehachse (ω) der Schraube (10) um einen Winkel (δ) zwischen 0 und 25 Grad, vorzugsweise zwischen 0 und 9 Grad, beabstandet ist.

6. Wirbelsäulenimplantat (1) nach einem der vorstehenden Ansprüche **dadurch gekennzeichnet, dass** die Bohrung (7) eine Rotationsachse (Ω) aufweist, die mit einer horizontalen Medianebene (πh) des Käfigs einen Winkel (α) zwischen 0 und 45 Grad, vorzugsweise zwischen 20 und 40 Grad, ausbildet.

7. Wirbelsäulenimplantat (1) nach einem der vorstehenden Ansprüche **dadurch gekennzeichnet, dass** dieses mehrere Schrauben (10) und mehrere Bohrungen (7), vorzugsweise drei Schrauben (10) und drei Bohrungen (7), umfasst.

8. Wirbelsäulenimplantat (1) nach einem der vorstehenden Ansprüche **dadurch gekennzeichnet, dass** der Käfig (2) eine oder mehrere Aussparungen umfasst, die den Formen einer spezifischen Zusatzstruktur entsprechen, um ein Greifen des Käfigs sicherzustellen.

9. Wirbelsäulenimplantat (1) nach Anspruch 1 **dadurch gekennzeichnet, dass** die Schraube (10) mindestens einen Kopf (11) und einen Hauptkörper (20) umfasst, worin der männliche Blockierbereich durch den Kopf (11) ausgebildet ist und die Wände (72) der Bohrung (7) mobile Bereiche umfassen, die eine Anpassung des Umfangs der Bohrung (7) ermöglichen, und ausgestaltet sind, während des Schraubens die relative Elastizität zwischen dem männlichen Blockierbereich und dem weiblichen Blockbereich bereitzustellen.

10. Wirbelsäulenimplantat (1) nach einem der vorstehenden Ansprüche **dadurch gekennzeichnet, dass** die Elemente des Wirbelsäulenimplantates (1) aus Materialien wie Titan, PEEK, PEKK, Kohlefaser oder einem resorbierfähigen Material gefertigt sind.

11. Wirbelsäulenimplantat (1) nach einem der vorstehenden Ansprüche **dadurch gekennzeichnet, dass** der Käfig (2) aus einem Titan-basierten Material mit Poren gefertigt ist, die ausgestaltet sind, die Austauschoberfläche zwischen dem Käfig (2) und der Außenseite des Käfigs (2) zu erhöhen, worin die Poren durch die Titan-Laser-Schmelztechnik erhalten werden.

12. Wirbelsäulenimplantat (1) nach einem der vorstehenden Ansprüche **dadurch gekennzeichnet, dass** dieses einen Höhenanpasser (100) zum Anpassen einer Höhe des Käfigs (2) umfasst.

13. Wirbelsäulenimplantat (1) nach dem vorstehenden Anspruch, **dadurch gekennzeichnet, dass** der Höhenanpasser (100) ein Verlängerungselement (103) umfasst, das nahe einer horizontalen Medianebene (πh) des Käfigs (2) angeordnet ist und einen oberen Bereich (101) und einen unteren Bereich (102) des Käfigs (2) trennt, wobei die Aktivierung des Verlängerungselementes (103) ermöglicht, den oberen Bereich (101) von dem unteren Bereich (102) weg zu bewegen und ersteren in einer gewünschten Entfernung von letzterem zu halten, um die Höhe des Käfigs (2) anzupassen.

14. Wirbelsäulenimplantat (1) nach einem der vorstehenden Ansprüche, **dadurch gekennzeichnet, dass** der Käfig (2) modular ist und mehrere trennbare Module umfasst.

15. Wirbelsäulenimplantat (1) nach dem vorstehenden Anspruch, **dadurch gekennzeichnet, dass** der Käfig (2) mindestens zwei trennbare Module umfasst, einschließlich eines oberen Modules (110), das einen männlichen Verbinder (112) umfasst und eines unteren Modules, (111) das einen weiblichen Verbinder (113) umfasst, worin der weibliche Verbinder (113) ausgestaltet ist, mit dem männlichen Verbinder (112) zusammenzuwirken, um das obere Modul (110) mit dem unteren Modul (111) zusammenzufügen, um den Käfig (2) auszubilden.

16. Wirbelsäulenimplantat (1) nach dem vorstehenden Anspruch, **dadurch gekennzeichnet, dass** der männliche Verbinder (112) ein L-förmiges Gleitelement und der weibliche Verbinder (113) eine L-förmige Rille umfasst, deren Form komplementär zu der des L-förmigen Gleitelementes ist, worin das Gleitelement (112) und die Rille (113) ausgestaltet sind, gleitend miteinander zusammenzuwirken, so dass der Aufbau des unteren Moduls (111) und des oberen Moduls (110) durch eine Gleitwirkung abgeschlossen wird.

17. Wirbelsäulenimplantat (1) nach einem der vorstehenden Ansprüche, **dadurch gekennzeichnet, dass** das Wirbelsäulenimplantat (1) implantierbar ist, vorzugsweise zwischen zwei Wirbelsäulenknochen des Patienten, vorzugsweise in einer zervikalen oder lumbalen Höhe.

18. Wirbelsäulenimplantat (1), welches entlang eines intersomatischen Raumes eines Patienten implantierbar ist und eine intersomatische Platte (120) und mindestens eine Fixierschraube (10) umfasst, die ausgestaltet ist, durch eine Bohrung (7) der Platte (120) in die Knochenmasse geschraubt zu werden, um die Position der Platte (120) entlang des intersomatischen Raumes zu stabilisieren, worin die Bohrung (7) und die Schraube (10) ein System zum Selbstblockieren der Schraube (10) in der Bohrung (7) ausbilden, worin das System einen mit der Schraube (10) einstückigen männlichen Blockierbereich und einen durch die Wände (72) der Bohrung (7) ausgebildeten weiblichen Blockbereich umfasst, worin der männliche Blockierbereich und der weibliche Blockbereich mit einer relativen Elastizität befestigt sind, die während des Einschraubens der Schraube (10) in die Knochenmasse durch die Bohrung (7) ein fortlaufendes und gegenseitiges Belasten des männlichen Blockierbereiches und des weiblichen Blockbereiches ermöglichen, bis eine finale Blockierposition der Schraube (10) erreicht ist, in welcher der männliche Blockierbereich und der weibliche Blockbereich gegenseitig entlastet sind, worin der männliche Blockierbereich durch den weiblichen Blockbereich in Position gesichert ist, worin die Schraube (10) mindestens einen Kopf (11) und einen Hauptkörper (20) umfasst, wobei der weibliche Blockbereich befestigt ist und der männliche Blockierbereich mobil ist und ein zurückziehbares System zum Anpassen des Umfanges des Kopfes (11) umfasst, das während des Schraubens die relative Elastizität zwischen dem männlichen Blockierbereich und dem weiblichen Blockbereich ermöglicht, wobei der männliche Blockierbereich und der weibliche Blockbereich mit einer relativen Elastizität befestigt sind, die während des Abschraubens der Schraube (10) aus der finalen Position ein gegenseitiges Belasten des männlichen Blockierbereiches und des weiblichen Blockbereiches ermöglicht, bis der männliche Blockierbereich von dem weiblichen Blockbereich abgeschraubt ist.

19. Wirbelsäulenimplantat (1) nach Anspruch 18, **dadurch gekennzeichnet, dass** die Wände (72) der Bohrung (7), von einer Öffnung (71) der Bohrung (7) und in die Schraubrichtung eine erste Fläche der Verringerung des Bohrungsquerschnittes (7) bis zu einer Schulter (73) ausbilden, bis zu welcher der Bohrungsquerschnitt (7) ansteigt und eine zweite Fläche definiert, worin die erste Fläche die radiale zentripetale Kompression des zurückziehbaren Systems ermöglicht, worin die zweite Fläche die radiale zentrifugale Ausdehnung des zurückziehbaren Systems ermöglich, worin die Schulter (73) zudem einen runden Lagerort (74) ausbildet, der senkrecht zu der Schraubrichtung, und ausgestaltet ist, mit dem zurückziehbaren System in Kontakt zu kommen, wenn die Schulter (73) in radialer Ausdehnung ist.

20. Wirbelsäulenimplantat (1) nach einem der Ansprüche 18 bis 19, **dadurch gekennzeichnet, dass** das zurückziehbare System durch mindestens einen Längsstreifen (14) ausgebildet ist, der mit seiner Basis (15) mit dem Kopf (11) verbunden ist und daher mindestens ein sich radial über den Kopf (11) erstreckendes Blatt ausbildet, worin der mindestens eine Streifen (14) elastisch mobil befestigt ist und eine elastische Ruheposition aufweist.

21. Wirbelsäulenimplantat (1) nach dem vorstehenden Anspruch, **dadurch gekennzeichnet, dass** die Schraube (10) drei Längsstreifen (14) umfasst, die über dem Kopf (11) angeordnet sind.

22. Wirbelsäulenimplantat (1) nach einem der vorstehenden Ansprüche, **dadurch gekennzeichnet, dass** in der elastischen Ruheposition, der Längsstreifen (14) von einer Drehachse (ω) der Schraube (10) um einen Winkel (δ) zwischen 0 und 25 Grad, vorzugsweise zwischen 0 und 9 Grad, beabstandet ist.

23. Wirbelsäulenimplantat (1) nach einem der Ansprüche 18 bis 22, **dadurch gekennzeichnet, dass** dieses mehrere Schrauben (10) und mehrere Bohrungen (7), vorzugsweise drei Schrauben (10) und drei Bohrungen (7), umfasst.

24. Wirbelsäulenimplantat (1) nach Anspruch 18, **dadurch gekennzeichnet, dass** die Schraube (10) mindestens einen Kopf (11) und einen Hauptkörper (20) umfasst, worin der männliche Blockierbereich durch den Kopf (11) ausgebildet ist und die Wände (72) der Bohrung (7) mobile Bereiche umfassen, die eine Anpassung des Umfangs der Bohrung (7) ermöglichen, und ausgestaltet sind, während des Schraubens die relative Elastizität zwischen dem männlichen Blockierbereich und dem weiblichen Blockbereich bereitzustellen.

25. Wirbelsäulenimplantat (1) nach einem der Ansprüche 18 bis 24, **dadurch gekennzeichnet, dass** die Bohrung (7) ein inneres Gewinde (121) umfasst, das mit einem Kopfgewinde (122) der Schraube zusammenwirken soll, worin das Kopfgewinde (122) an dem Schraubenkopf (11) vorgesehen ist.

26. Medizinisches Kit, das durch ein Wirbelsäulenimplantat (1) nach einem der vorstehenden Ansprüche und einer Zusatzstruktur zum Setzen des Wirbelsäulenimplantates in oder entlang des intersomatischen Raumes eines Patienten gebildet ist.

## Claims

1. A vertebral implant (1) intended to be implanted in an intersomatic space of a patient and comprising an intersomatic cage (2) and at least one fixing screw (10) intended to be screwed into the bony mass through a well (7) of said cage (2) to stabilize the position of said cage (2) within the intersomatic space, said well (7) and the screw (10) forming a system for the self-locking of the screw (10) in the well (7), said system comprising a male locking portion integral with the screw (10) and a female lock portion formed by the walls (72) of the well (7), said male locking portion and female lock portion being mounted with a relative flexibility allowing, during the screwing of the screw (10) into the bony mass through the well (7), a progressive and mutual stressing of the male locking portion and female lock portion until reaching a final locking position of the screw (10) in which the male locking portion and female lock portion are mutually released, the male locking portion being locked in position by the female lock portion, said screw (10) comprising at least a head (11) and a main body (20), said female lock portion being fixed and said male locking portion being mobile and including a retractable system for modifying the circumference of said head (11) allowing, during the screwing, the relative flexibility between said male locking portion and female lock portion, said male locking portion and female lock portion being mounted with a relative flexibility allowing, during the unscrewing of the screw (10), from said final position, a mutual stressing of the male locking portion and female lock portion until unlocking the male locking portion from the female lock portion.

2. The vertebral implant (1) according to any of the preceding claims, **characterized in that** the walls (72) of the well (7) form, from an orifice (71) of the well (7) and in the screwing direction, a first area of reduction of the cross-section of the well (7), said first area allowing the radial centripetal compression of said retractable system, up to a shoulder (73) increasing the cross-section of the well (7) and defining a second area allowing the radial centrifugal expansion of said retractable system, said shoulder (73) also forming a circular bearing place (74), perpendicular to the screwing direction, and being configured to be in contact with said retractable system when said shoulder (73) is in radial expansion.

3. The vertebral implant (1) according to any of the preceding claims, **characterized in that** said retractable system is formed by at least one longitudinal tab (14), connected by its base (15) to said head (11) and hence forming at least one petal extending radially about said head (11), said at least one tab (14) being mounted elastically mobile and having an elastic rest position.

4. The vertebral implant (1) according to any of the preceding claims **characterized in that** said screw (10) includes three longitudinal tabs (14) arranged about its head (11).

5. The vertebral implant (1) according to any of the two preceding claims **characterized in that**, in the elastic rest position, the longitudinal tab (14) is spaced apart from an axis of rotation (ω) of the screw (10) by an angle (δ) between 0 and 25 degrees, preferably between 0 and 9 degrees.

6. The vertebral implant (1) according to any of the preceding claims **characterized in that** said well (7) has an axis of revolution (Ω) that forms an angle (α) between 0 and 45 degrees, preferably between 20 and 40 degrees, with a horizontal median plane (πₕ) of said cage.

7. The vertebral implant (1) according to any of the preceding claims **characterized in that** it includes several screws (10) and several wells (7), preferably three screws (10) and three wells (7).

8. The vertebral implant (1) according to any of the preceding claims **characterized in that** said cage (2) includes one or several notches matching the shapes of a specific ancillary so as to ensure a gripping of the cage.

9. The vertebral implant (1) according to claim 1 **characterized in that** said screw (10) comprises a least a head (11) and a main body (20), said male locking portion being formed by said head (11) and the walls (72) of the well (7) including mobile portions allowing a modification of the circumference of the well (7) configured to provide, during the screwing, said relative flexibility between said male locking portion and female lock portion.

10. The vertebral implant (1) according to any of the preceding claims **characterized in that** the vertebral implant's (1) elements are made from materials such as titanium, PEEK, PEKK, carbon fibres or a resorbable material.

11. The vertebral implant (1) according to any of the preceding claims **characterized in that** said cage (2) is made from a titanium-based material having pores configured to increase the surface of exchange between the cage (2) and the outside of the cage (2), said pores being obtained by the titanium laser melting technique.

12. The vertebral implant (1) according to any of the preceding claims **characterized in that** it comprises a height adjuster (100) for adjusting a height of the cage (2).

13. The vertebral implant (1) according to the preceding claim, **characterized in that** the height adjuster (100) includes an elongation element (103) disposed near a horizontal median plane (πh) of the cage (2) and separating an upper portion (101) and a lower portion (102) of the cage (2), the activation of the elongation element (103) allowing to move the upper portion (101) away from the lower portion (102) and to hold it at a desired distance from the latter, so as to vary the height of said cage (2).

14. The vertebral implant (1) according to any of the preceding claims, **characterized in that** said cage (2) is modular and comprises a plurality of separable modules.

15. The vertebral implant (1) according to the preceding claim, **characterized in that** said cage (2) comprises at least two separable modules including an upper module (110) comprising a male connector (112) and a lower module (111) comprising a female connector (113), said female connector (113) being designed to cooperate with the male connector (112) so as to assemble the upper module (110) with the lower module (111) to form said cage (2).

16. The vertebral implant (1) according to the preceding claim, **characterized in that** the male connector (112) comprises a L-shaped slide, the female connector (113) comprising a L-shaped groove, whose shape is complementary to that of the L-shaped slide, the slide (112) and the groove (113) being designed to slidingly cooperate with each other, so that the assembly of the lower module (111) and the upper module (110) is made by a sliding action.

17. The vertebral implant (1) according to any of the preceding claims, **characterized in that** the vertebral implant (1) is implantable, preferably between two bones of the patient's vertebral column, preferably at a cervical or lumbar level.

18. A vertebral implant (1) implantable along an intersomatic space of a patient and comprising an intersomatic plate (120) and at least one fixing screw (10) configured to be screwed into the bony mass through a well (7) of said plate (120) to stabilize the position of said plate (120) along the intersomatic space, the well (7) and the screw (10) forming a system for the self-locking of the screw (10) in the well (7), said system comprising a male locking portion integral with the screw (10) and a female lock portion formed by the walls (72) of the well (7), said male locking portion and female lock portion being mounted with a relative flexibility allowing, during the screwing of the screw (10) into the bony mass through the well (7), a progressive and mutual stressing of the male locking portion and female lock portion until reaching a final locking position of the screw (10) in which the male locking portion and female lock portion are mutually released, the male locking portion being locked in position by the female lock portion, said screw (10) comprising at least a head (11) and a main body (20), said female lock portion being fixed and said male locking portion being mobile and including a retractable system for modifying the circumference of said head (11) allowing, during the screwing, the relative flexibility between said male locking portion and female lock portion, said male locking portion and female lock portion being mounted with a relative flexibility allowing, during the unscrewing of the screw (10), from said final position, a mutual stressing of the male locking portion and female lock portion until the male locking portion is unscrewed from the female lock portion.

19. The vertebral implant (1) according to claim 18, **characterized in that** the walls (72) of the well (7) form, from an orifice (71) of the well (7) and in the screwing direction, a first area of reduction of the cross-section of the well (7), said first area allowing the radial centripetal compression of said retractable system, up to a shoulder (73) increasing the cross-section of the well (7) and defining a second area allowing the radial centrifugal expansion of said retractable system, said shoulder (73) also forming a circular bearing place (74), perpendicular to the screwing direction, and being configured to be in contact with said retractable system when said shoulder (73) is in radial expansion.

20. The vertebral implant (1) according to any of claims 18 to 19, **characterized in that** said retractable system is formed by at least one longitudinal tab (14), connected by its base (15) to said head (11) and hence forming at least one petal extending radially about said head (11), said at least one tab (14) being mounted elastically mobile and having an elastic rest position.

21. The vertebral implant (1) according to the preceding claim, **characterized in that** said screw (10) includes three longitudinal tabs (14) arranged about its head (11).

22. The vertebral implant (1) according to any of the two preceding claims, **characterized in that** in the elastic rest position, the longitudinal tab (14) is spaced apart from an axis of rotation (ω) of the screw (10) by an angle (δ) between 0 and 25 degrees, preferably between 0 and 9 degrees.

23. The vertebral implant (1) according to any of claims 18 to 22, **characterized in that** it includes several screws (10) and several wells (7), preferably three screws (10) and three wells (7).

24. The vertebral implant (1) according to claim 18, **characterized in that** said screw (10) comprises a least a head (11) and a main body (20), said male locking portion being formed by said head (11) and the walls (72) of the well (7) including mobile portions allowing a modification of the circumference of the well (7) configured to provide, during the screwing, said relative flexibility between said male locking portion and female lock portion.

25. The vertebral implant (1) according to any of claims 18 to 24, **characterized in that** the well (7) includes an an inner thread 121 intended to cooperate with a head thread (122) of the screw, said head thread (122) being located at the screw head (11).

26. A medical kit formed by a vertebral implant (1) according to any of the preceding claims and an ancillary for the setting of said vertebral implant in or along the intersomatic space of a patient.
